# EUROPEAN PATENT SPECIFICATION

(11) **EP 2 370 017 B1**
(45) Date of publication and mention of the grant of the patent: **02.12.2015**
(21) Application number: 09771635.1
(22) Date of filing: 10.12.2009
(51) Int. Cl.: A61B 19/00, A61M 5/178, A61M 29/02

(54) **CLIP FOR HANDLING AN ENDOSCOPIC DEVICE**
KLAMMER ZUR HANDHABUNG EINER ENDOSKOPVORRICHTUNG
FIXATION POUR MANIPULER UN DISPOSITIF ENDOSCOPIQUE

(30) Priority: 11.12.2008 US 121815 P; 08.12.2009 US 633483
(43) Date of publication of application: 05.10.2011
(73) Proprietor: Boston Scientific Scimed, Inc., Maple Grove, MN 55311-1566 (US)
(72) Inventor: RYAN, Shawn, Upton Massachusetts 01568 (US); PACKET, Nick, Brighton Massachusetts 02135 (US)
(74) Representative: Vossius & Partner Patentanwälte Rechtsanwälte mbB
(86) International application number: PCT/US2009/067475
(87) International publication number: WO 2010/068739

(56) References cited:
- WO-A1-89/00409
- WO-A1-99/67156
- US-A- 5 027 478
- US-A- 5 364 355
- US-B1- 6 869 417

## Description

### Background

Needle biopsies are often performed to diagnose and/or stage diseases. In these procedures, an endoscope may be placed into a gastrointestinal tract or other lumen of a living body (e.g., via a naturally occurring body orifice). Once a target tissue mass has been identified (e.g., visually using a vision system of an endoscope), an endoscopic ultrasound guided fine-needle aspiration ('EUS-FNA") device is introduced into a working channel of the endoscope with a stylet positioned to occlude a distal opening of the needle to prevent non-targeted tissue from entering the lumen. The stylet is withdrawn from the distal opening of the needle when the needle is positioned adjacent to the target tissue mass and the needle is inserted into the target tissue to capture a sample of target tissue therein.

US 5 364 355 A discloses an intraluminal system which includes an elongated, flexible, intraluminal device, such as a catheter or a guidewire, with means on the intraluminal device to releasably secure one or more turns of a coiled flexible product. The coiled flexible product may be a coiled portion of the intraluminal device on which the securing means is mounted or it may be a separate coiled product. The securing function is performed by means of one or more flexible arms on a body directly or indirectly secured to the intraluminal device so that, when it is desired to remove the coiled product, it can be manually disengaged from the securing means without a great deal of force. The securing means is adapted to hold at least one turn of an elongated coiled product and preferably a plurality of turns so that the coiled product can be maintained in a compact arrangement.

US 5 027 478 A relates to a coiling clamp for linear flexible materials which is formed as a unitary plastic, metal or wooden body member having circumferentially spaced outwardly opening receiving recesses which may be of different sizes to receive different sizes of the linear material such as garden hose, electrical extension cords, water-ski ropes and the like.

US 6 869 417 B1 is directed to an attachment tool for facilitating the attachment of an elongated instrument like a catheter having a connector thereon to an elongated guide member like a guide-wire. The tool comprises surfaces arranged to cause the catheter to contact the guide-wire upon the application of a force, e.g., a twisting force, a longitudinal force, a lateral force, etc., to the tool or to the catheter, whereupon the catheter is attached to the guide wire,

WO 89/00409 A1 relates to a guidewire gripping device which may be attached to a medical guJdewire. The guidewire gripping device comprises an elongratedr, cylindrical body made of an elastic material. The body includes an elongated slot which extends into the body from its circumference. The slot is adapted to receive a guidewire. The device also includes handles attached to either side of the elongated slot. Force applied to the handles causes the slot to open for insertion of the guidewire. The internal sides of the slot act to frictionally grip the fuiedeiwre.

Finally, WO 99/67156 A1 describes a container for use in angiographic procedures or other medical procedures such as endoscopic procedures utilizing an elongated, flexible medical device. The container comprises a base, and side walls which extend upwardly and inwardly from the base. The container is open at the top so that a coiled guide wire can be placed inside the container. The cross-sectional area of the opening is smaller than the cross-sectional area of the base, and the opening and the base may be of complementary shape.

### Summary of the invention

The present invention is directed to a device for storing a stylet comprising a stylet sized and shaped for insertion into a lumen of a needle an end cap selectively coupleable to a proximal end of a needle, the end cap being coupled to a proximal end of the stylet so that, when the end cap is coupled to the needle, the stylet extends from the end cap through the lumen of the needle, the end cap including a clip mechanism for gripping a distal portion of the stylet when the end cap is decoupled from the needle, the clip mechanism including a space through which the distal end of the stylet may pass substantially parallel to the proximal end thereof.

### Brief Description of the Drawings

Fig. 1 shows a partial exploded view of an FNA device comprising an end cap according to a first exemplary embodiment of the present invention;
Fig. 2 shows a perspective view of an end cap according to a second exemplary embodiment of the present invention;
Fig. 3 shows a perspective view of an end cap according to a third exemplary embodiment of the present invention;
Fig. 4 shows a perspective view of an end cap according to a fourth exemplary embodiment of the present invention;
Fig. 5 shows a perspective view of an end cap according to a fifth exemplary embodiment of the present invention;
Fig. 6 shows a perspective view of an end cap according to a sixth exemplary embodiment of the present invention;
Fig. 7 shows a perspective view of an end cap according to a seventh exemplary embodiment of the present invention;
Fig. 8 shows a perspective view of an end cap according to an eighth exemplary embodiment of the present invention; and
Fig. 9 shows a perspective view of an end cap according to a ninth exemplary embodiment of the present invention.

### Detailed Description

The present invention, which may be further understood with reference to the following description and the appended drawings, relates to devices for conducting biopsy procedures and in particular, to EUS-FNA biopsy needles. Exemplary embodiments of the present invention provide a device and method by which a physician or other use of an EUS-FNA device can manipulate a stylet at all stages of a biopsy procedure.

An exemplary embodiment according to the present invention comprises an end cap which may be employed with any FNA device, endoscope or other device inserted into a living body to perform a medical procedure. The end cap is provided with a retention mechanism to grip and temporarily lock a stylet in place. In this manner, the stylet remains controlled and easily accessible to the physician and may be easily removed from and reinserted into a needle multiple times in a single biopsy procedure as needed.

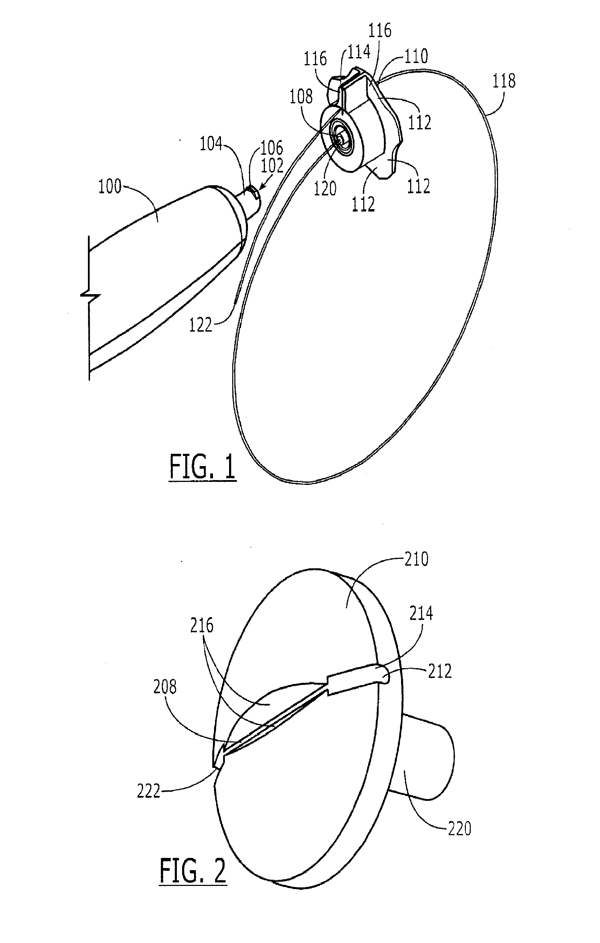

As shown in Fig. 1, an FNA device 100 according to an exemplary embodiment of the invention comprises a lumen 102 extending therethrough from a proximal end which remains external to the body at all times to a distal end (not shown) which, when in an operative position, is inserted into the body to a location adjacent to a target tissue mass to be sampled. The FNA device 100 comprises a connector (e.g., a male luer 104) at a proximal end thereof. This connector is sized and shaped to engage a corresponding connector (e.g., a female luer 108) of an end cap 110. The male luer 104 in this embodiment includes threading 106 on a proximal end thereof enabling the male luer 104 to be screwed into the female luer 108 to lock the end cap to the device 100 as those skilled in the art will understand. Alternatively, the end cap 110 may simply be pushed into a proximal end of the FNA device 100 and held in engagement therewith by a substantial friction fit or any other known connection. As would be understood by those skilled in the art, the end cap 110 may also include a series of grooves 112 forming ergonomic gripping surfaces facilitating manipulation thereof (e.g., screwing and unscrewing the end cap 110 from the device 100). Furthermore, as would be understood by those skilled in the art, the end cap 110 may be formed of any known material having the desired strength and rigidity such as plastic injection molded or shaped by any another known method. The end cap 110 includes a stylet 118 formed, for example, as a flexible wire sized to slidably pass through the lumen 102 and to seal a distal opening in the needle (not shown). A proximal end of the stylet 118 is permanently mounted in a retainer 120 (e.g., using any known attachment method such as adhesive, over-molding of the retainer, etc.) which, when the end cap 110 is mounted on the device 100 is aligned with the lumen 102. In an alternate embodiment, the retainer 120 may be formed as an opening extending through the end cap 110 and open at a proximal end thereof. The stylet 118 in this embodiment is not bonded to the retainer 120 and may be removed from the proximal opening to permit a user to manipulate the stylet 118 without removing the end cap 110 from the FNA device 100. Furthermore, the stylet 118 may comprise an enlarged proximal end (not shown) to prevent a full insertion into the lumen 102, as those skilled in the art will understand. The retainer 120 may, for example, be formed as a projection from the distal side of the end cap 110 which slides into the male luer 104 when the end cap 110 is mounted on the device 100. The end cap 110 may also include a slot 114 extending radially into the end cap 110 from an outer perimeter thereof, as shown in Fig. 1. The slot 114 is formed as an opening bordered on lateral sides by walls 116 and open at a radially outer end to a radially outer edge of the end cap 110. The slot 114 extends through an entire thickness of the end cap 110 from a proximal side to a distal side thereof and a width of the slot is selected to receive the stylet 118 therein (e.g., with a friction fit).

In use, a distal end of the stylet 118 is inserted into the lumen 102 until the distal end of the stylet 118 plugs a distal opening in the needle (not shown) and the device 100 is inserted to a target site within the body. When the distal end of the needle has reached the target site, the user unscrews the end cap 110 from the male luer 104 and withdraws the stylet from the lumen 102. The stylet 118 may then be coiled and a distal portion thereof may be slid into the groove 114. As indicated above, the walls 116 may apply a substantial friction force to the stylet 118 to maintain a position thereof and prevent the stylet 118 from uncoiling or otherwise becoming dislodged from the end cap 110. Then, if it is desired to move the needle to another location (e.g., to sample a separate tissue mass or another portion of the initial target tissue mass), the stylet 118 may be removed from the groove 114 and reinserted into the lumen 102 to close the distal end of the needle, preventing non-targeted tissue from entering the lumen 102 as the needle is inserted through intervening tissue to the second target location. The stylet 118 may then be removed from the lumen 102, coiled and gripped in the groove 114 while a tissue sample from the second target site is retrieved. This process may then be repeated as many times as desired, significantly reducing the effort required to store and reinsert the stylet 118 for each new target site. The exemplary embodiment of the present invention allows for single handed manipulation of the stylet 118 with minimal deviation from the standard hand motion required to remove the stylet 118, and thus does not further complicate the FNA biopsy procedure.

As shown in Fig. 2, an end cap 210 according to an alternate embodiment of the invention is constructed substantially similarly to the end cap 110 except that groove 214 of the end cap 210 extends laterally across a proximal surface of the end cap 210 and does not extend through a thickness thereof (i.e., through the end cap 210 from a proximal to a distal side thereof) as with the groove 114. A stylet (not shown) extends from the distal side of the end cap 210 in the same manner shown for the end cap 110. Similarly to the groove 114, the groove 214 may be sized and shaped to provide a friction fit to retain the stylet therein with a substantial friction fit, via a pair of flexible flaps extending across the groove or via any other known mechanism as would be understood by those skilled in the art. The groove 214 will hold the stylet therein until a predetermined minimum force is applied to remove the stylet therefrom. The end cap 210 is also provided with a retainer 220 which serves as a proximal joint for the stylet (not shown). The end cap 210 is also formed to mate to the proximal end of a corresponding needle (not shown) in the same manner described above in regard to the end cap 110.

In use, the stylet is inserted into the lumen of a needle to which the end cap 210 is to be mounted until the stylet plugs the distal opening of the needle. The end cap 210 is then mounted to the proximal end of the needle in the same manner described above for the end cap 110 and the device 100. The needle is then inserted through intervening tissue to a target site adjacent a tissue mass to be sampled. When the target site has been reached, the user removes the end cap 210 from the proximal end of the needle and withdraws the stylet from the lumen. The stylet may then be coiled and a distal portion thereof is inserted between beveled walls 216 and into the groove 214. As would be understood by those skilled in the art, the beveled walls 216 are angled away from one another and provide a wider entry to the groove 214 which aids in aiming the stylet into the groove 214. The beveled walls 216 may extend toward one another to a distance less than a diameter of the groove 214 and less than an outer diameter of the stylet so that, the stylet is retained within the groove 214 by the beveled walls 216. The end cap 210 or a portion forming the beveled walls 216 may be formed of a material with sufficient flexibility to allow the stylet 118 to be pushed past the reduced size opening between beveled walls 216 and snapped into the groove 214, as those skilled in the art will understand. When the user wishes to remove the stylet from the end cap 210, ends of the stylet projecting beyond the ends of the groove 214 may be grasped and the gripped portion of the stylet may be popped out of the groove 214 by applying a distally directed force to the end cap 210 while holding the stylet in place. In this manner, the stylet may be slid out of the end cap 210 and reinserted into the needle so that the needle may be moved through intervening tissue to a second target site without capturing non-targeted tissue. The end cap 210 may then be removed so that the stylet may be withdrawn from the needle for the capture of a tissue sample from the second target site. This process may then be repeated as often as desired. It is also envisioned that the stylet may be slid longitudinally out of the groove 214 by pulling on the portion of the stylet extending proximally of the groove 214. It is further noted that the end cap 210 may be modified to include one of ergonomic grooves and another gripping means to facilitate screwing the end cap 210 on and off the needle (or to assist in any other manipulations of the end cap 210) without deviating from the scope of the present invention.

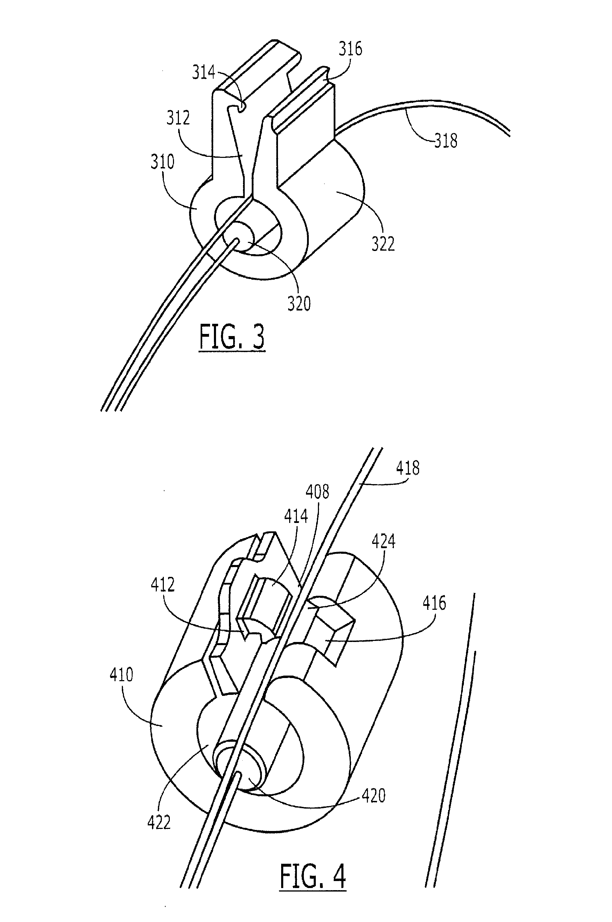

As shown in Fig. 3, an end cap 310 according to another embodiment of the invention comprises a retainer 320 bonded to a proximal end of a stylet 318. The end cap 310 comprises a latch design formed of a substantially flexible material movable between an open configuration in which a hook 314 is separated from a latch 316, as shown in Fig. 3, to expose an opening 312 into which the stylet 318 may be inserted and a locked configuration closing the opening 312 to lock the stylet therein, as will be described in more detail below. The hook 314 is movable manually over the latch 316 to move the end cap 310 to the locked configuration. As would be understood by those skilled in the art, the hook 314 may extend over the side of the latch 316 by a depth selected to prevent inadvertent movement of the end cap 310 to the open configuration. As can be seen in Fig. 3, the latch 316 and the hook 314 are separated from one another along a line substantially parallel to a longitudinal axis of a needle to which the end cape 310 will be coupled (i.e., parallel to a direction of the proximal part of the stylet 318 extending therefrom. The opening 312 extends parallel to this axis as well and opens to a slot sized to receive the stylet therewithin. As would be understood by those skilled in the art, the latch 316 and the hook 314 may include any desired complimentary geometries to enhance the locking of the end cap 310 in the locked configuration. It is further noted that the retainer 320 may be secured to the end cap 310 by any known attachment method (e.g., adhesive, welding, etc.). In the exemplary embodiment shown, an outer diameter of the circular portion 322 of the end cap 310 is approximately 10.67 mm but may vary depending on the requirements of a procedure to be performed.

As shown in Fig. 4, an end cap 410 according to another embodiment of the invention, is manually movable from an open configuration in which a tab 414 is separated from a recess 416 to form an opening 422 and a closed configuration in which the tab 414 is latched into the recess 416. When in the locked configuration, the tab 414 is separated radially from a retainer 420 bonded to the proximal end of the stylet 418 by a gap 408 the size of which is selected to permit the stylet 418 to rest therewithin. As with the previously described embodiments, the end cap 410 is formed as a substantially cylindrical element with an opening 422 extending longitudinally therethrough (i.e., parallel to a longitudinal axis of the needle to which the end cap 410 is to be coupled. However, as would be understood by those skilled in the art, the end cap may be made any desired shape so long as it mates with the proximal end of a needle to which it is to be coupled in a desired manner and the openings which receive the distal portions of the stylet in any of these embodiments may be oriented in any desired manner. The orientation parallel to the proximal portion of the stylet is shown as it facilitates coiling of the stylet but is not required. The tab 414 may be manually pressed over the recess 416 and held in place (i.e., in the locked configuration) via engagement of an abutting portion 412 of the tab 414 with a protruding portion 424 of the recess 416. Specifically, the end cap 410 may be biased to the open configuration shown in Fig. 4 with a latch at the end of the abutting portion 412 locking against an angled surface of the protruding portion 424 with a radial expansion pressure applied by the abutting portion 412 onto the protruding portion 424 maintaining the end cap 410 in the locked position. To return the end cap 410 to an open configuration, the suer manually applies a further radial constriction force to move the abutting portion 412 of the tab 414 out of engagement with the protruding portion 424 of the recess 416.

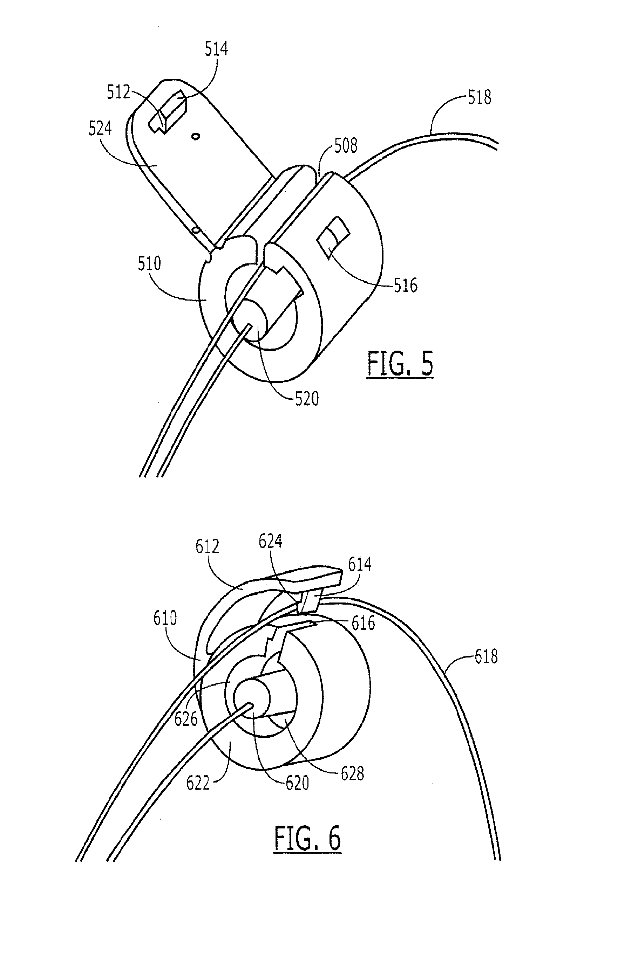

As shown in Fig. 5, an end cap 510 according to another embodiment of the invention also comprises a retainer 520 permanently attached thereto and receiving a proximal end of a stylet 518. The end cap 510 includes an outer perimeter extending radially around the retainer 520 with a slot 508 extending longitudinally therethrough (proximally to distally). An arm 524 with a tab 514 extending radially inward from a radially inner surface thereof is pivotally mounted to the outer perimeter of the end cap 510. The arm 524 is sized so that, when rotated to a locked position, the arm 524 extends across the slot 508 with the tab 514 received within a latch 516 formed on a portion of the outer perimeter of the end cap 510. In the open configuration shown, the slot 508 is exposed permitting a distal portion of the stylet 518 to be inserted therethrough to enter a central opening 522 of the end cap 510. The material of which the arm 524 (or any desired portions thereof) and the pivotal connection between the arm 524 and the outer perimeter of the end cap 510 may be selected to be more flexible than a material of which the rest of the end cap 510 is formed. This may be employed to facilitate the bending of the arm 524 around the outer perimeter of the end cap 510 to the locked position. However, the arm 524 may be formed with a bias toward to open position to facilitate manual opening of the slot 508 when desired. A user may push the arm 524 radially inward until the tab 514 comes into engagement with the latch 516 locking the arm 524 in the locked position as would be understood by those skilled in the art. As with the end cap 410 of Fig. 4, the end cap 510 may be retained in the locked position by a radial expansion pressure applied through the bias of the arm 524 via an abutting portion 512 of the tab 514 to the latch 516. Movement back to the open position requires manual application of a further radial constriction force to the end cap 510 to move the tab 514 out of engagement with the latch 516, as also indicated above.

As shown in Fig. 6, an end cap 610 according to another embodiment of the invention includes a flexible arm 612 pivotally extending from an outer perimeter of the end cap 610 for movement between an open configuration exposing a slot 616 in the outer perimeter and a locked configuration in which the arm 612 is latched over the slot 616. Similarly to the end cap 510 described above, the arm 612 is preferably made sufficiently flexible to permit the bending of the arm 612 repeatedly between the open and locked configurations while retaining a desired bias toward the open configuration to apply a locking force between a notch 624 formed on a tab 616 and a groove 626 formed within the slot 616. The end cap 610 is provided with a retainer 620 receiving the proximal end of a stylet 618 therein in the same manner described above for the other embodiments. The retainer 620 may be permanently joined to the end cap 610 along a distal face thereof, which, as shown in Fig. 6, is provided with a seal 628. In this case, the distal portion of the stylet 618 is inserted radially within the arm 612 and the outer perimeter of the end cap 610 (i.e., in a gap therebetween) while the tab 624 is latched into the slot 616. In the same manner described above for the previous embodiments, the end cap 610 may be coupled to the proximal end of a needle (e.g., by screwing thereonto).

In use, a distal portion of the stylet 618 is inserted into a needle (not shown) in the same manner thereof and the end cap 610 is coupled to the proximal end of the needle. The needle is then inserted through intervening tissue to a target site adjacent to a portion of tissue to be sampled. The user then removes the end cap 610 from the needle and withdraws the stylet from the lumen of the needle and moves the needle into the target tissue mass to capture a sample. The user stores the stylet by coiling it and inserting the distal end thereof between the arm 612 and the outer perimeter of the end cap 610 and moves the arm 612 to the locked position. The sample may then be removed from the needle in any known manner (e.g., via suction in the lumen of the needle). The user then moves the arm 612 back to the open configuration and removes the distal end of the stylet from the end cap 610 and reinserts the distal end of the stylet into the needle. The user then recouples the end cap 610 to the needle and moves the needle to a second target site. The user may then remove the end cap 610 from the needle and withdraw and store the stylet (i.e., coiling the stylet and locking the distal end thereof between the arm 612 and the outer perimeter of the end cap 610) to capture a second sample. This process may be repeated as often as desired without withdrawing the needle from the body.

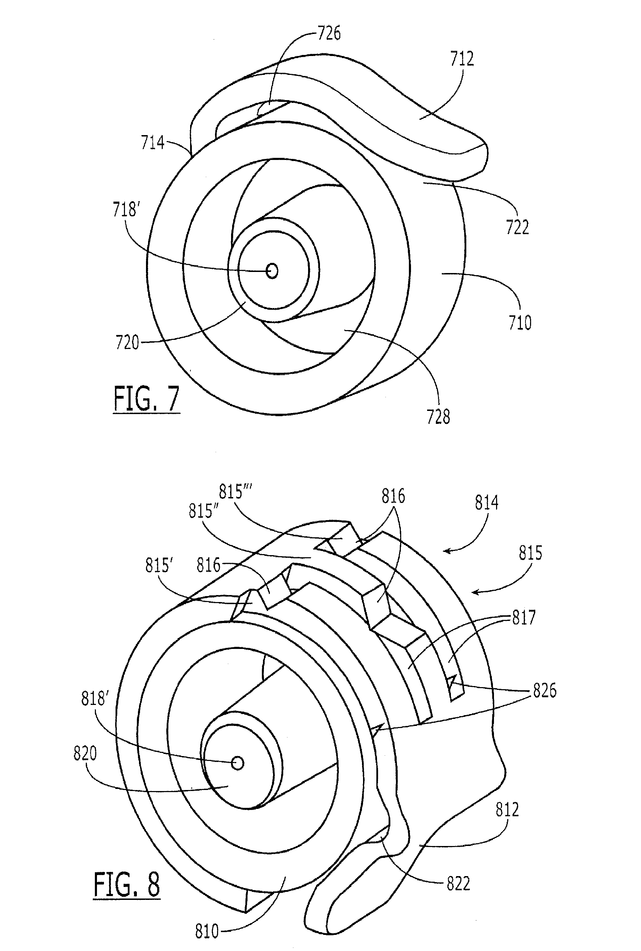

As shown in Fig. 7, an end cap 710 according to yet another embodiment of the present invention includes an arm 712 integrally formed therewith. Specifically, a first portion of the arm 712 extends from a first end 714 around a portion of an outer perimeter of the end cap 710 separated from the outer perimeter by a space 726. A second portion of the arm 712 extends from the first portion toward the outer perimeter to close an opening 722 to the space 726. Those skilled in the art will understand that the second portion of the arm 712 need not contact the outer perimeter of the end cap 710 to close opening 722. Rather, the second portion of the arm 712 need only reduce a separation between a radially inner surface of the arm 712 and the outer perimeter at the opening 722 to a distance less than a diameter of a stylet to be inserted into the space 726. Similarly to the end cap 610, the arm 712 is preferably sufficiently flexible to permit bending of the arm 712 to temporarily increase the size of the space 726 to permit a stylet (not shown) to be slid therethrough. When the stylet has been positioned therein as desired, the arm 712 may be released to return under natural bias to its original position gripping the stylet between the arm 712 and the outer perimeter of the end cap 710. Alternatively, a user may slide the stylet laterally into the space 726 via the opening 722 by pressing the stylet between the radially inner surface of the arm 712 and the outer perimeter of the end cap 710 to bend the arm 712 away from the outer perimeter until the stylet enters the space 726. At this point, the arm 712 will spring back to the closed position by the natural bias of the material and the stylet will be locked within the space 726. The end cap 710 includes a retainer 720 coupled to the proximal end of the stylet in the same manner described above for the other embodiments. As with the previously described embodiments, the retainer 720 may be permanently joined to the end cap 710 along a distal face thereof, which, as shown in Fig. 7, is also provided with a seal 728. The end cap 710 is shown including a recess 718' through which the stylet projects.

In use, a distal portion of the stylet is inserted into a needle (not shown) in the same manner discussed above with respect to Fig. 6. The end cap 710 is coupled to the proximal end of the needle and the needle is advanced to a target site adjacent to a portion of tissue to be sampled. The user then removes the end cap 710 from the needle and withdraws the stylet from the lumen of the needle and moves the needle into the target tissue mass to capture a sample. The user stores the stylet by coiling it and inserting the distal end thereof into the space 726 as described above. When the user wishes to remove the stylet from the end cap 710, ends of the stylet projecting beyond the ends of the opening 722 may be grasped and the gripped portion of the stylet may be popped out of the opening 722 by applying a lateral force to the end cap 710 while holding the stylet in place. In this manner, the stylet may be slid out of the end cap 710 and reinserted into the needle so that the needle may be moved through intervening tissue to a second target site without capturing non-targeted tissue. It is also envisioned that the stylet may be slid longitudinally out of the space 726 by pulling on the portion of the stylet extending proximally of the space 726.

As shown in Fig. 8, an end cap 810 according to yet another embodiment of the present invention is constructed substantially similarly to the end cap 710 except that the arm 812 of the end cap 810 lies flush against an outer perimeter of the end cap 810 without defining a space corresponding to the space 726 between a radially inner surface of the arm 812 and the outer perimeter of the end cap 810 to receive a stylet. Unlike the embodiment of Fig. 7, the arm 812 is not formed integrally with the end cap 810 but is formed as a separate member attached thereto. Specifically, the arm 812 is formed as a partially circular element extending over a portion of a perimeter of the end cap 810 (i.e., more than half the circumference of the end cap 810) with a natural bias of the material thereof clamping the arm 812 around the end cap 810. The arm 812 is preferably formed of a material sufficiently rigid to prevent inadvertent movement from a closed configuration in which an entire perimeter of the arm 812 lies flush against the end cap 810 to an open configuration in which a portion of the arm 812 is bent radially away from the outer perimeter of the end cap 810. The arm 812 includes a living hinge 814 formed as a series of strips 815 of material extending circumferentially around the end cap 810 and separated from one another by slits 817 with each of the strips 815 defining one or more areas of enhanced flexibility as will be described in more detail below. The length and location of the living hinge 814 is chosen to permit bending along one side of the partially circular arm 812. The strips 815 are formed integrally with the arm 812 and further define portions of bendability therein.

Each strip 815 comprises a first groove 816 formed along a radially outer face of the arm 812. The first groove 816 comprises beveled walls angling away from one another from a joint lying on the strip 815. As shown in the embodiment of Fig. 8, the first grooves 816 are situated along the length of the strips 815 at locations chosen to achieve a desired flexibility and a location at which bending will occur.

Specifically, the first grooves 816 may be disposed on the strips 815 in an alternating pattern, with a first strip 815' comprising a first groove 816 at a first position (e.g., a predetermined distance along the length of the living hinge 814) and a second adjacent strip 815" including a first groove 816 at a second position (e.g., a second predetermined distance along the length of the living hinge 814) different from the first length. This distribution pattern may then be repeated so that a third strip 815"' comprises a first groove 816 at the first position and so on. It is noted that, although the living hinge 814 shown comprises three strips 815, any number of strips 815 may be employed without deviating from the spirit and scope of the present invention.

The first strip 815' and the third strip 815"' also comprise second grooves 826 formed along an inner surface of the arm 812. The second grooves 826 comprise beveled walls angling away from one another from a joint lying on the radially inner face of the arm 812, wherein the bevel angle is smaller than the bevel angle of the first grooves 816. As shown in Fig. 8, the second grooves 826 are placed along the first strip 815' and the third strip 815'" at a positions substantially opposite the first grooves 816. As those skilled in the art will understand, the placement of the first and second grooves 816, 826 facilitates radially outward bending of the arm 812, thus allowing a user to easily remove and reattach the arm 812 from the end cap 810. Specifically, the first and second grooves 816, 826 absorb the stress applied to the arm 812 when bending radially outward to move to the open configuration, as described above, wherein the greater bevel of the first grooves 816 permits a greater flexion in this direction. It is noted that although the exemplary embodiment shown comprises strips 815 including first and second grooves 816, 826, any number of grooves may be employed at any number of locations without deviating from the spirit and scope of the present invention. It is preferable, however, that the number of first grooves 816 be offset by a respective ratio of second grooves 816 to reduce the likelihood of fracture of the arm 812.

The arm 812 may be further provided with a recess 822 defined by a convex portion of the arm 812. The recess 822 is sized to receive a portion of a stylet (not shown) therein. Specifically, after the end cap 810 has been removed from a needle, the arm 812 may be manually moved from the closed configuration to the open configuration and locked into the closed configuration again by, for example, a radially constrictive bias preformed into the arm 812 during manufacturing. As with earlier embodiment, the end cap 810 also comprises a retainer 820 with an opening 818' adapted to couple to a proximal end of the stylet (not shown).

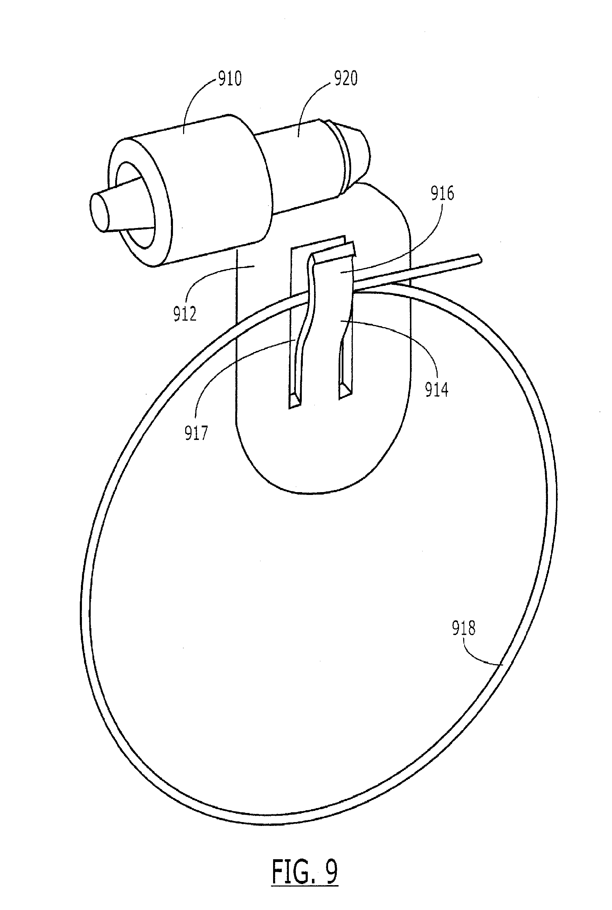

Fig. 9 depicts yet another alternate embodiment of the present invention wherein a retainer for receiving a distal end of a stylet 918 is formed as a tab extending from an end cap 910. Specifically, a proximal face of the end cap 910 is provided with a link 920 comprising a tab 912 extending radially outward therefrom. The link 920 may be shaped substantially similarly to the end cap 910 (e.g., cylindrical) and is rotatably attached to the end cap 910 permitting a user to selectively position the tab 912 at any desired angle with respect to the end cap 910. The tab 912 further comprises a clip 914 formed as a cut-out. A substantially "U" shaped slit 917 defines the shape of the clip 914, which is further formed with a convex portion 916 sized and shape to receive a proximal portion of a stylet 918 to be held therein. In use, after a needle (not shown) has been traversed to a target site in the body, the end cap 910 may be removed from engagement with a proximal end of the needle, as described in greater detail in earlier embodiments. The stylet 918 may then be removed from the needle and a proximal portion of the stylet 918 may be pushed into the convex portion 916 of the clip 914 to be held therein via friction applied thereto by the clip 914.

It is noted that the devices and components of Figs. 1 - 9 can be modified or combined in any of a number of ways without deviating from the spirit and scope of the present invention. For example, any of the disclosed embodiments may be modified to comprise a living hinge in the end cap. Furthermore, the clip function may be integrated elsewhere on an FNA device or endoscope and does not necessarily need to be housed in a stylet end cap. In one example, the clip feature may be formed integrally with a proximal portion of the FNA device itself. Alternatively, the clip may be sized and shape to be slidably received over a shaft of an FNA device or endoscope.

The end cap and clipping arrangement of the present invention may be employed with any medical device for retaining a stylet and are not restricted to the embodiments shown in Figs. 1 - 9. For example, the end cap and clip may employ one or more of a friction fit, snap fit and interference fit to engage a distal portion of a stylet. It will therefore be apparent to those skilled in the art that various modifications and variations may be made to the structure and methodology of the present invention without departing from the scope of the invention. Thus, the present invention covers all modifications and variations so as they come within the scope of the appended claims and their equivalents.

## Claims

1. A system comprising:
a needle (100) having a lumen (102) extending therethrough from a proximal end which is configured to remain external to a body to a distal end which is configured to be inserted into said body to a location adjacent to a target tissue mass to be sampled, wherein the needle (100) comprises a connector (104) at the proximal end thereof; and
a device for storing a stylet (118) comprising:
a stylet (118);
an end cap (110) having a connector (108) selectively coupleable to the connector (104) at the proximal end of the needle (100), the end cap (110) being coupled to a proximal end of the stylet (118) so that, when the end cap (110) is coupled to the needle (100), the stylet (118) extends from the end cap (110) through the lumen (102) of the needle (100); the end cap (110) including a clip mechanism for gripping a distal portion of the stylet (118) when the end cap (110) is decoupled from the needle (100), the clip mechanism including a space (114) through which a distal portion of the stylet (118) may pass substantially parallel to the proximal end thereof.

2. The system according to claim 1, wherein the end cap (110) is formed integrally with the stylet (118).

3. The system according to claim 1, wherein the clip mechanism is a cutout of a wall of the end cap (110).

4. The system according to claim 1, wherein the stylet (118) is sized and shaped for insertion into the lumen (102) of the needle (100), the needle (100) adapted for aspiration.

5. The system according to claim 1, wherein the clip mechanism comprises a slot (114) formed in the end cap sized and shaped to frictionally engage the stylet (118).

6. The system of claim 5, wherein edges of the slot (114) are flexible to permit deformation as the stylet (118) is inserted therein.

7. The system of claim 5, wherein the slot (114) is formed between two walls (116) extending through a thickness of the end cap (110) from a proximal face to a distal face thereof.

8. The system of claim 1, wherein the clip mechanism includes an arm (712) movable around a portion of an outer surface of the end cap (710) to lock the stylet (118) between the arm (712) and the outer surface of the end cap (710).

9. The system of claim 8, wherein the arm (712) comprises a first portion extending parallel to the outer surface of the end cap (710) separated therefrom by an annular space (726) and a second portion extending toward the outer surface to close a lateral opening thereto.

10. The system of claim 9, wherein a thickness of the annular space (726) is equal to a thickness of the stylet (118) and wherein a radially inner surface of the second portion of the arm (712) is separated from the outer perimeter by a space a thickness of which is less than the thickness of the stylet (118), and wherein the radially inner surface of the second portion of the arm (712) abuts the outer surface of the end cap (710).

11. The system of claim 8, wherein the arm (524) comprises a hook and wherein a latch (516) is formed on a portion of the outer surface of the end cap (510) over which the hook is located when in a locked configuration.

12. The system of claim 1, wherein the clip mechanism includes a living hinge (814).

13. The system of claim 1, wherein the clip mechanism includes a tab (912) that extends radially outward from a link (920) of the end cap (910).

14. The system of claim 13, wherein the space (917) of the clip mechanism is defined by a convex portion (916) of the tab (912).

## Patentansprüche

1. System, das aufweist:
eine Nadel (100) mit einem Lumen (102), das sich durch sie von einem proximalen Ende, das so konfiguriert ist, dass es außerhalb eines Körpers bleibt, zu einem distalen Ende erstreckt, das so konfiguriert ist, dass es in den Körper zu einer Stelle benachbart zu einer zu beprobenden Zielgewebemasse eingeführt wird, wobei die Nadel (100) einen Konnektor (104) an ihrem proximalen Ende aufweist; und
eine Vorrichtung zum Aufbewahren eines Stiletts (118), die aufweist:
ein Stilett (118);
eine Endkappe (110) mit einem Konnektor (108), der mit dem Konnektor (104) am proximalen Ende der Nadel (100) selektiv koppelbar ist, wobei die Endkappe (110) mit einem proximalen Ende des Stiletts (118) so gekoppelt ist, dass sich bei Kopplung der Endkappe (110) mit der Nadel (100) das Stilett (118) von der Endkappe (110) durch das Lumen (102) der Nadel (100) erstreckt; wobei die Endkappe (110) einen Klemmenmechanismus zum Ergreifen eines distalen Abschnitts des Stiletts (118) aufweist, wenn die Endkappe (110) von der Nadel (100) entkoppelt ist, und der Klemmenmechanismus einen Raum (114) aufweist, der von einem distalen Abschnitt des Stiletts (118) im Wesentlichen parallel zu seinem proximalen Abschnitt durchlaufen werden kann.

2. System nach Anspruch 1, wobei die Endkappe (110) integral mit dem Stilett (118) ausgebildet ist.

3. System nach Anspruch 1, wobei der Klemmenmechanismus ein Ausschnitt einer Wand der Endkappe (110) ist.

4. System nach Anspruch 1, wobei das Stilett (118) zur Einführung in das Lumen (102) der Nadel (100) bemessen und geformt ist, wobei die Nadel (100) zur Aspiration geeignet ist.

5. System nach Anspruch 1, wobei der Klemmenmechanismus einen in der Endkappe gebildeten Schlitz (114) aufweist, der so bemessen und geformt ist, dass er einen reibschlüssigen Eingriff mit dem Stilett (118) herstellt.

6. System nach Anspruch 5, wobei Kanten des Schlitzes (114) flexibel sind, um Verformung zu ermöglichen, wenn das Stilett (118) darin eingeführt wird.

7. System nach Anspruch 5, wobei der Schlitz (114) zwischen zwei Wänden (116) gebildet ist, die sich durch eine Dicke der Endkappe (110) von einer proximalen Fläche zu einer distalen Fläche davon erstrecken.

8. System nach Anspruch 1, wobei der Klemmenmechanismus einen Arm (712) aufweist, der um einen Abschnitt einer Außenfläche der Endkappe (710) beweglich ist, um das Stilett (118) zwischen dem Arm (712) und der Außenfläche der Endkappe (710) zu verriegeln.

9. System nach Anspruch 8, wobei der Arm (712) aufweist: einen ersten Abschnitt, der sich parallel zur Außenfläche der Endkappe (710) erstreckt, der durch einen ringförmigen Raum (726) davon getrennt ist, und einen zweiten Abschnitt, der sich zur Außenfläche erstreckt, um eine Seitenöffnung zu ihr zu schließen.

10. System nach Anspruch 9, wobei eine Dicke des Ringraums (726) gleich einer Dicke des Stiletts (118) ist und wobei eine Radialinnenfläche des zweiten Abschnitts des Arms (712) vom Außenumfang durch einen Raum getrennt ist, dessen Dicke kleiner als die Dicke des Stiletts (118) ist, und wobei die Radialinnenfläche des zweiten Abschnitts des Arms (712) an der Außenfläche der Endkappe (710) anliegt.

11. System nach Anspruch 8, wobei der Arm (524) einen Haken aufweist und wobei ein Riegel (516) auf einem Abschnitt der Außenfläche der Endkappe (510) gebildet ist, über dem der Haken in einer verriegelten Konfiguration liegt.

12. System nach Anspruch 1, wobei der Klemmenmechanismus ein Filmscharnier (814) aufweist.

13. System nach Anspruch 1, wobei der Klemmenmechanismus eine Lasche (912) aufweist, die sich von einem Verbindungsstück (920) der Endkappe (910) radial nach außen erstreckt.

14. System nach Anspruch 13, wobei der Raum (917) des Klemmenmechanismus durch einen konvexen Abschnitt (916) der Lasche (912) gebildet ist.

## Revendications

1. Système comprenant :
une aiguille (100) ayant une lumière (102) s'étendant à travers cette dernière, à partir d'une extrémité proximale qui est configurée pour rester à l'extérieur d'un corps, jusqu'à une extrémité distale qui est configurée pour être insérée dans ledit corps jusqu'à un emplacement adjacent à une masse de tissu cible à échantillonner, dans lequel l'aiguille (100) comprend un connecteur (104) au niveau de son extrémité proximale ; et
un dispositif pour stocker un stylet (118) comprenant :
un stylet (118) ;
un capuchon d'extrémité (110) ayant un connecteur (108) pouvant être couplé sélectivement au connecteur (104) au niveau de l'extrémité proximale de l'aiguille (100), le capuchon d'extrémité (110) étant couplé à une extrémité proximale du stylet (118) de sorte que, lorsque le capuchon d'extrémité (110) est couplé à l'aiguille (100), le stylet (118) s'étend à partir du capuchon d'extrémité (110) à travers la lumière (102) de l'aiguille (100) ; le capuchon d'extrémité (110) comprenant un mécanisme d'attache pour saisir une partie distale du stylet (118) lorsque le capuchon d'extrémité (110) est découplé de l'aiguille (100), le mécanisme d'attache comprenant un espace (114) à travers lequel une partie distale du stylet (118) peut passer sensiblement parallèlement par rapport à son extrémité proximale.

2. Système selon la revendication 1, dans lequel le capuchon d'extrémité (110) est formé de manière solidaire avec le stylet (118).

3. Système selon la revendication 1, dans lequel le mécanisme d'attache est une découpe d'une paroi du capuchon d'extrémité (110).

4. Système selon la revendication 1, dans lequel le stylet (118) est dimensionné et formé pour l'insertion dans la lumière (102) de l'aiguille (100), l'aiguille (100) étant adaptée pour l'aspiration.

5. Système selon la revendication 1, dans lequel le mécanisme d'attache comprend une fente (114) formée dans le capuchon d'extrémité, dimensionnée et formée pour mettre en prise le stylet (118) par friction.

6. Système selon la revendication 5, dans lequel les bords de la fente (114) sont flexibles pour permettre la déformation lorsque le stylet (118) est inséré à l'intérieur de cette dernière.

7. Système selon la revendication 5, dans lequel la fente (114) est formée entre deux parois (116) s'étendant à travers une épaisseur du capuchon d'extrémité (110), d'une face proximale jusqu'à sa face distale.

8. Système selon la revendication 1, dans lequel le mécanisme d'attache comprend un bras (712) mobile autour d'une partie d'une surface externe du capuchon d'extrémité (710) pour verrouiller le stylet (118) entre le bras (712) et la surface externe du capuchon d'extrémité (710).

9. Système selon la revendication 8, dans lequel le bras (712) comprend une première partie s'entendant parallèlement à la surface externe du capuchon d'extrémité (710) séparée de cette dernière par un espace annulaire (726) et une seconde partie s'étendant vers la surface externe pour fermer une ouverture latérale par rapport à cette dernière.

10. Système selon la revendication 9, dans lequel une épaisseur de l'espace annulaire (726) est égale à une épaisseur du stylet (118) et dans lequel une surface radialement interne de la seconde partie du bras (712) est séparée du périmètre externe par un espace, dont une épaisseur est inférieure à l'épaisseur du stylet (118), et dans lequel la surface radialement interne de la seconde partie du bras (712) vient en butée contre la surface externe du capuchon d'extrémité (710).

11. Système selon la revendication 8, dans lequel le bras (524) comprend un crochet et dans lequel un verrou (516) est formé sur une partie de la surface externe du capuchon d'extrémité (510) sur lequel le crochet est positionné lorsqu'il est dans la configuration verrouillée.

12. Système selon la revendication 1, dans lequel le mécanisme d'attache comprend une charnière active (814).

13. Système selon la revendication 1, dans lequel le mécanisme d'attache comprend une languette (912) qui s'étend radialement vers l'extérieur à partir d'une liaison (920) du capuchon d'extrémité (910).

14. Système selon la revendication 13, dans lequel l'espace (917) du mécanisme d'attache est défini par une partie convexe (916) de la languette (912).
